# FASCICULE DE BREVET EUROPEEN

(11) **EP 4 197 450 B1**
(45) Date de publication et mention de la délivrance du brevet: **21.01.2026**
(21) Numéro de dépôt: 22211465.4
(22) Date de dépôt: 05.12.2022
(51) Int. Cl.: A61B 8/02, A61B 8/06, A61B 8/08, A61B 8/00

(54) **DISPOSITIF ET PROCEDE D'EVALUATION D'ACTIVITE CARDIOVASCULAIRE PAR ULTRASONS**
VORRICHTUNG UND VERFAHREN ZUR BEURTEILUNG VON KARDIOVASKULÄREN AKTIVITÄTEN UNTER VERWENDUNG VON ULTRASCHALL
DEVICE AND METHOD FOR ASSESSING CARDIOVASCULAR ACTIVITY USING ULTRASOUND

(30) Priorité: 20.12.2021 FR 2113944
(43) Date de publication de la demande: 21.06.2023
(73) Titulaire: Vermon, 37000 Tours (FR)
(72) Inventeur: BENCHEMOUL, Maxime, 37000 TOURS (FR)
(74) Mandataire: Cabinet Beaumont

(56) Documents cités:
- FR-A1- 2 759 892
- US-A1- 2004 138 568
- US-A1- 2019 069 842

## Description

### Domaine technique

La présente description concerne de façon générale les dispositifs électroniques, et vise plus particulièrement des dispositifs et des procédés d'évaluation d'activité cardiovasculaire par ultrasons.

### Technique antérieure

On connaît des dispositifs électroniques et des procédés d'évaluation d'activité cardiovasculaire permettant, par exemple, de détecter des dysfonctionnements susceptibles d'affecter un système cardiovasculaire d'un être humain, de surveiller un état clinique d'un patient atteint de maladie cardiovasculaire ou de suivre une évolution de paramètres cardiovasculaires d'un athlète au cours d'une performance sportive.

Le document FR 2759892 décrit un système d'échographie ultrasonore pour l'examen des artères. La demande de brevet US 2004/138568 A1 décrit un dispositif électronique avec deux sondes à ultrasons pour l'évaluation du pouls d'un utilisateur, ledit dispositif étant au poignet.

### Résumé de l'invention

Un objet d'un mode de réalisation est de pallier tout ou partie des inconvénients des dispositifs et des procédés connus d'évaluation d'activité cardiovasculaire. Plus particulièrement, un objet d'un mode de réalisation est de fournir un dispositif électronique d'évaluation d'activité cardiovasculaire portable, et présentant des dimensions extérieures compatibles avec un port au poignet d'un utilisateur.

L'invention est définie par la revendication 1. Les revendications dépendantes couvrent des modes de réalisation et variantes de l'invention.

### Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :
la figure 1 est une vue de dessus illustrant, de façon schématique et partielle, un exemple d'un dispositif électronique portable d'évaluation d'activité cardiovasculaire selon un mode de réalisation ;
la figure 2 est une vue en coupe, selon le plan AA de la figure 1, du dispositif de la figure 1 dans une première configuration d'utilisation ;
la figure 3 est une vue en coupe, selon le plan AA de la figure 1, du dispositif de la figure 1 dans une deuxième configuration d'utilisation ;
la figure 4 représente, de façon schématique et partielle, un exemple de réalisation d'un circuit de commande du dispositif de la figure 1 ;
la figure 5 représente, de façon schématique et partielle, un autre exemple de réalisation du circuit de commande du dispositif de la figure 1 ; et
la figure 6 illustre un exemple d'intégration du dispositif de la figure 1 dans une montre intelligente portée au poignet d'un utilisateur.

### Description des modes de réalisation

De mêmes éléments ont été désignés par de mêmes références dans les différentes figures. En particulier, les éléments structurels et/ou fonctionnels communs aux différents modes de réalisation peuvent présenter les mêmes références et peuvent disposer de propriétés structurelles, dimensionnelles et matérielles identiques.

Par souci de clarté, seuls les étapes et éléments utiles à la compréhension des modes de réalisation décrits ont été représentés et sont détaillés. En particulier, les diverses applications des dispositifs et des procédés décrits n'ont pas été détaillées, les modes de réalisation décrits étant compatibles avec toutes ou la plupart des applications susceptibles de tirer profit d'un dispositif ou d'un procédé d'estimation d'activité cardiovasculaire. En outre, les transducteurs ultrasonores des sondes du dispositif n'ont pas été détaillés, la réalisation et la mise en œuvre pratique de ces transducteurs étant à la portée de la personne du métier à partir des indications de la présente description.

Sauf précision contraire, lorsque l'on fait référence à deux éléments connectés entre eux, cela signifie directement connectés sans éléments intermédiaires autres que des conducteurs, et lorsque l'on fait référence à deux éléments reliés (en anglais "coupled") entre eux, cela signifie que ces deux éléments peuvent être connectés ou être reliés par l'intermédiaire d'un ou plusieurs autres éléments.

Dans la description qui suit, lorsque l'on fait référence à des qualificatifs de position absolue, tels que les termes "avant", "arrière", "haut", "bas", "gauche", "droite", etc., ou relative, tels que les termes "dessus", "dessous", "supérieur", "inférieur", etc., ou à des qualificatifs d'orientation, tels que les termes "horizontal", "vertical", etc., il est fait référence, sauf précision contraire, à l'orientation des figures.

Sauf précision contraire, les expressions "environ", "approximativement", "sensiblement", et "de l'ordre de" signifient à 10 % près, de préférence à 5 % près ou, lorsqu'il s'agit d'une valeur angulaire, à 10° près, de préférence à 5° près.

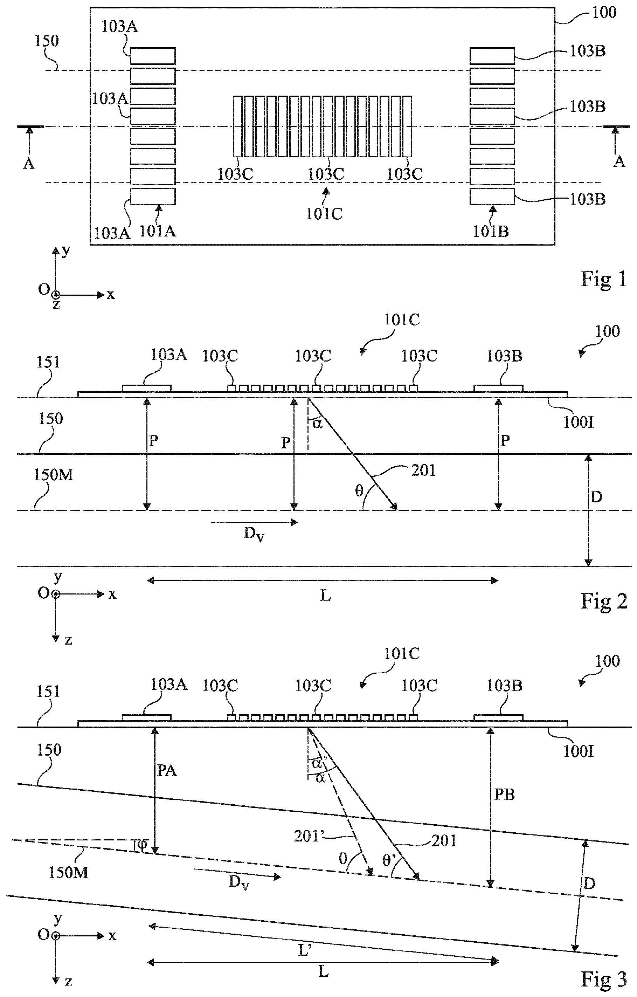

La figure 1 est une vue de dessus illustrant, de façon schématique et partielle, un exemple d'un dispositif électronique portable 100 d'évaluation d'activité cardiovasculaire par ultrasons selon un mode de réalisation.

Dans l'exemple représenté, le dispositif 100 comprend des première, deuxième et troisième sondes 101A, 101B, 101C comportant chacune une barrette de transducteurs ultrasonores 103A, respectivement 103B, respectivement 103C. Dans l'exemple représenté, les barrettes de transducteurs 103A, 103B et 103C sont sensiblement linéaires. À titre de variante, chaque barrette de transducteurs 103A, 103B et 103C peut présenter une forme non rectiligne, par exemple courbe. En outre, on a illustré en figure 1 un exemple dans lequel les transducteurs 103A, 103B, 103C d'une même barrette présentent des dimensions extérieures sensiblement identiques, aux dispersions de fabrication près, et sont régulièrement espacés. Toutefois, en variante, les transducteurs 103A, 103B, 103C d'une même barrette peuvent présenter des dimensions différentes les uns des autres et un espacement variable.

Les première et deuxième sondes ultrasonores 101A, 101B sont situées de part et d'autre de la troisième sonde ultrasonore 101C et sont sensiblement parallèles entre elles. La troisième sonde 101C est sensiblement perpendiculaire aux première et deuxième sondes 101A, 101B. Dans cet exemple, les première, deuxième et troisième sondes 101A, 101B, 101C présentent, en vue de dessus, une forme générale en H, les première et deuxième sondes 101A, 101B formant respectivement les deux barres verticales du H et la troisième sonde 101C formant la barre horizontale du H.

Dans l'exemple illustré en figure 1, les sondes 101A, 101B comportent chacune huit transducteurs ultrasonores 103A, 103B et la sonde 101C comporte seize transducteurs ultrasonores 103C. Toutefois, les sondes 101A, 101B, 101C peuvent comporter des nombres de transducteurs ultrasonores 103A, 103B, 103C différents de ceux représentés. À titre d'exemple, le nombre de transducteurs ultrasonores 103A, 103B, 103C de chaque barrette correspond à une puissance de deux et le nombre de transducteurs ultrasonores 103C de la sonde centrale 101C est deux fois plus important que le nombre de transducteurs ultrasonores 103A, 103B de chacune des deux sondes latérales 101A, 101B. Les transducteurs ultrasonores 103A, 103B, 103C présentent par exemple chacun une forme sensiblement rectangulaire. En outre, les transducteurs 103C peuvent être moins larges que les transducteurs 103A, 103B de sorte que la sonde 101C présente une résolution latérale plus importante que les sondes 101A, 101B, par exemple dans un cas où la sonde 101C est utilisée en mode B, ou pour effectuer un tir Doppler défléchi électroniquement, et les sondes 101A, 101B sont utilisées en mode A comme cela sera exposé plus en détail par la suite.

Dans l'exemple représenté, le dispositif électronique portable 100 est disposé au-dessus d'un vaisseau sanguin 150, par exemple une artère, dont une paroi est symbolisée, en figure 1, par deux traits en pointillé. À des fins de simplification, on néglige l'épaisseur du vaisseau sanguin 150, les deux traits en pointillé de la figure 1 pouvant ainsi symboliser indifféremment les parois internes ou externes du vaisseau sanguin 150. Dans cet exemple, les barrettes de transducteurs ultrasonores 103A, 103B des première et deuxième sondes 101A, 101B sont orientées transversalement par rapport au vaisseau sanguin 150, et la barrette de transducteurs ultrasonores 103C de la troisième sonde 101C est orientée longitudinalement par rapport au vaisseau sanguin 150. Plus précisément, dans l'exemple illustré, la barrette de transducteurs ultrasonores 103C est sensiblement parallèle à un axe Ox, le long duquel s'étend latéralement le vaisseau sanguin 150, tandis que les barrettes de transducteurs ultrasonores 103A, 103B sont sensiblement parallèles à un axe Oy, perpendiculaire à l'axe Ox. À titre d'exemple, dans un cas où le dispositif 100 est porté au bras ou au poignet d'un utilisateur, le vaisseau sanguin 150 est l'artère ulnaire.

À des fins de simplification, on a représenté en figure 1 un exemple dans lequel le vaisseau sanguin 150 est sensiblement rectiligne et présente une section de forme, par exemple circulaire, et de dimensions sensiblement constantes sur toute sa longueur. Toutefois, en pratique, le vaisseau sanguin 150 peut présenter une forme quelconque, par exemple une forme courbe, et une section de forme et de dimensions variables sur tout ou partie de sa longueur. En outre, on a représenté en figure 1 un exemple dans lequel les barrettes de transducteurs ultrasonores 103A, 103B, 103C sont sensiblement centrées par rapport au vaisseau sanguin 150. Cet exemple n'est toutefois pas limitatif, les barrettes de transducteurs ultrasonores 103A, 103B, 103C pouvant être décentrées, par exemple décalées suivant l'axe Oy, par rapport au vaisseau sanguin 150.

À des fins de lisibilité du dessin, on a illustré en figure 1 un exemple dans lequel le vaisseau sanguin 150 présente un diamètre supérieur à la longueur (dimension prise selon l'axe Oy) des transducteurs 103C. Le vaisseau sanguin 150 peut toutefois présenter des proportions différentes par rapport aux transducteurs 103C, par exemple un diamètre sensiblement égal à la longueur des transducteurs 103C, ou un diamètre inférieur à la longueur des transducteurs 103C.

La figure 2 est une vue en coupe, selon le plan AA de la figure 1, du dispositif électronique portable 100 d'évaluation d'activité cardiovasculaire humaine dans une première configuration d'utilisation.

Dans cette configuration d'utilisation, le vaisseau sanguin 150 est sensiblement parallèle à une face inférieure 100I sensiblement plane du dispositif 100. Plus précisément, le vaisseau sanguin 150 présente un axe médian 150M parallèle à l'axe Ox, la face inférieure 100I du dispositif 100 étant parallèle au plan Oxy. Dans l'exemple représenté, les transducteurs ultrasonores 103A, 103B, 103C sont sensiblement équidistants de l'axe médian 150M du vaisseau sanguin 150. Dit autrement, dans cet exemple, le vaisseau sanguin 150 s'étend latéralement sous les transducteurs ultrasonores 103A, 103B, 103C à une profondeur P sensiblement constante. Dans l'orientation de la figure 2, la profondeur P correspond à une distance, mesurée le long de l'axe vertical Oz, entre la face inférieure 100I du dispositif 100 et l'axe médian 150M du vaisseau sanguin 150. À titre d'exemple, la face inférieure 100I du dispositif 100 est placée sur et en contact avec la peau d'un bras 151 de l'utilisateur.

Les transducteurs ultrasonores 103A, 103B des sondes 101A, 101B du dispositif 100 permettent par exemple d'estimer un débit volumique Dᵥ de sang circulant à l'intérieur d'une portion du vaisseau sanguin 150 située sous le dispositif 100, par exemple par un procédé de détermination de vitesse d'onde de pouls ("Pulse Wave Velocity" - PWV, en anglais). La vitesse d'onde de pouls, notée VOP, est par exemple calculée à partir d'une mesure de temps de propagation d'onde de pouls ("Pulse Transit Time" - PTT, en anglais), produite à chaque battement cardiaque, entre les transducteurs ultrasonores 103A et 103B.

Pour cela, le dispositif 100 enregistre par exemple des évolutions temporelles DA(t), DB(t) d'un diamètre D du vaisseau sanguin 150 évalué en deux endroits distincts séparés d'une distance L, par exemple à l'aplomb des sondes 101A et 101B. Une mesure de déphasage Δt entre les évolutions temporelles DA(t), DB(t) du diamètre D, enregistrées respectivement grâce aux sondes 101A et 101B à la suite d'un même battement cardiaque, permet d'estimer le temps de propagation de l'onde de pouls entre ces sondes. Connaissant la distance L séparant les sondes 101A et 101B, on peut alors remonter à la vitesse de propagation de l'onde de pouls VOP à l'intérieur du vaisseau sanguin 150 (VOP = L/Δt).

À titre d'exemple, les variations temporelles DA(t), DB(t) du diamètre D du vaisseau sanguin 150 sont évaluées en mode A. Dans ce mode, on mesure une amplitude d'un signal d'écho renvoyé en fonction de la profondeur. À titre de variante, les variations temporelles DA(t), DB(t) du diamètre D du vaisseau sanguin 150 peuvent être évaluées à partir d'images réalisées en mode B. Dans ce mode, on réalise des images échographiques bidimensionnelles composées de points lumineux représentant des échos ultrasonores, la luminosité de chaque point étant déterminée par l'amplitude du signal d'écho renvoyé.

En outre, les transducteurs ultrasonores 103C de la troisième sonde 101C peuvent permettre d'estimer le débit volumique Dᵥ du sang circulant à l'intérieur de la portion du vaisseau sanguin 150 située sous le dispositif 100. L'estimation du débit Dᵥ par la sonde 101C est par exemple effectuée par effet Doppler.

Dans ce cas, les transducteurs ultrasonores 103C sont par exemple commandés pour émettre un faisceau ultrasonore incident 201 en direction du vaisseau sanguin 150, et pour recevoir un faisceau ultrasonore réfléchi par le sang circulant à l'intérieur du vaisseau sanguin 150. Le faisceau ultrasonore incident 201 correspond par exemple à un signal impulsionnel de fréquence fᵢ et le faisceau ultrasonore réfléchi correspond par exemple à un signal impulsionnel de fréquence fᵣ. En mesurant un décalage fréquentiel entre les signaux incident et réfléchi, c'est-à-dire en mesurant une différence entre les fréquences fᵣ et fᵢ, on peut remonter à la vitesse v de déplacement du sang à l'intérieur du vaisseau sanguin 150. En outre, les mesures du diamètre D permettent d'estimer une section S du vaisseau sanguin 150 sous le dispositif 100. La section S correspond par exemple à une surface d'une coupe transversale du vaisseau sanguin 150. En multipliant la section S du vaisseau sanguin 150 par la vitesse v d'écoulement du sang à travers cette section, on peut alors remonter au débit volumique Dᵥ du sang à l'intérieur du vaisseau sanguin 150 (Dᵥ = S*v).

À titre d'exemple, le diamètre D du vaisseau sanguin 150 est évalué, en mode A ou en mode B, par les transducteurs ultrasonores 103C de la sonde 101C. À titre de variante, le diamètre D du vaisseau sanguin peut être évalué, en mode A ou en mode B, par les transducteurs ultrasonores 103A de la sonde 101A et/ou par les transducteurs ultrasonores 103B de la sonde 101B comme exposé précédemment.

Lors de l'estimation du débit sanguin Dᵥ par effet Doppler, le faisceau ultrasonore incident 201 émis par la barrette de transducteurs ultrasonores 103C présente une direction d'émission formant par exemple un angle α, appelé angle de déflexion ("steering angle", en anglais), par rapport à une normale à la surface inférieure 100I du dispositif 100. Pour obtenir une précision de mesure optimale de la vitesse v, l'angle α est par exemple ajusté de sorte que le faisceau ultrasonore 201 atteigne le vaisseau sanguin 150 sous un faible angle d'incidence θ, appelé angle Doppler. À titre d'exemple, l'angle θ est compris entre 30° et 60°.

En outre, le faisceau ultrasonore incident 201 est par exemple focalisé au niveau de l'axe médian 150M du vaisseau sanguin 150, c'est-à-dire à la profondeur P sous le dispositif 100, dans cet exemple. Cela permet par exemple de mesurer la vitesse v au centre de l'écoulement et non à proximité des parois du vaisseau sanguin 150. Il en résulte une meilleure précision de mesure.

À titre de variante, la sonde 101C peut être utilisée pour réaliser de l'imagerie échographique en mode B, plus précisément de l'imagerie par ondes planes ("Plane Wave Imaging" - PWI, en anglais) à haute fréquence d'acquisition. À partir des images ainsi obtenues, le diamètre D du vaisseau sanguin 150 et la vitesse v de circulation du sang peuvent par exemple être estimés par une technique dite d'imagerie de flux vectoriel ("Vector Flow Imaging" - VFI, en anglais).

La figure 3 est une vue en coupe, selon le plan AA de la figure 1, du dispositif électronique portable 100 d'évaluation d'activité cardiovasculaire humaine dans une deuxième configuration d'utilisation.

Dans cette configuration d'utilisation, le vaisseau sanguin 150 est incliné par rapport au dispositif 100. Plus précisément, le vaisseau sanguin 150 est, dans cet exemple, parallèle au plan Oxz et incliné d'un angle φ par rapport à l'axe horizontal Ox, la face inférieure 100I du dispositif 100 étant parallèle au plan Oxy. Dans cet exemple, les transducteurs ultrasonores 103B de la deuxième sonde 101B sont plus éloignés de l'axe médian 150M du vaisseau sanguin 150 que les transducteurs ultrasonores 103A de la première sonde 101A. Plus précisément, dans l'exemple illustré en figure 3, l'axe médian 150M du vaisseau sanguin 150 est situé à une profondeur PA sous les transducteurs ultrasonores 103A et à une autre profondeur PB, supérieure à la profondeur PA, sous les transducteurs ultrasonores 103B. Dans l'orientation de la figure 3, les profondeurs PA, PB correspondent à des mesures de distance le long de l'axe vertical Oz.

Dans l'exemple illustré en figure 3, l'inclinaison du vaisseau sanguin 150 modifie l'angle d'incidence du faisceau ultrasonore incident 201 émis par la sonde 101C en direction du vaisseau sanguin 150. Plus précisément, dans cet exemple, le faisceau 201 atteint le vaisseau sanguin 150 sous un angle d'incidence θ' inférieur à l'angle θ (θ' = θ + φ). Cela tend à dégrader la précision de la mesure de la vitesse v, donc l'estimation du débit volumique Dᵥ du sang à l'intérieur du vaisseau sanguin 150.

En outre, dans l'exemple illustré en figure 3, l'angle φ d'inclinaison du vaisseau sanguin 150 tend à provoquer une focalisation du faisceau ultrasonore incident 201 émis par la sonde 101C dans une zone éloignée de l'axe médian 150M, c'est-à-dire plus proche des parois du vaisseau sanguin 150M que dans l'exemple illustré en figure 2. Cela tend également à dégrader la précision de la mesure de la vitesse v, donc du débit Dᵥ.

Selon un mode de réalisation, on tire profit des barrettes latérales de transducteurs ultrasonores 103A, 103B situées de part et d'autre de la barrette centrale de transducteurs ultrasonores 103C pour émettre des faisceaux ultrasonores permettant d'estimer la position du vaisseau sanguin 150 par rapport au dispositif 100. En fonction de la position estimée du vaisseau sanguin 150, le faisceau ultrasonore 201 émis par la troisième sonde 101C est ensuite ajusté.

Plus précisément, les transducteurs ultrasonores 103A émettent par exemple un premier faisceau ultrasonore permettant d'estimer la position, par exemple la profondeur PA le long de l'axe Oz, à laquelle le vaisseau sanguin 150 se situe à l'aplomb de la sonde 101A. De façon analogue, les transducteurs ultrasonores 103B émettent par exemple un deuxième faisceau ultrasonore permettant de déterminer la position, par exemple la profondeur DB le long de l'axe Oz, à laquelle le vaisseau sanguin 150 se situe à l'aplomb de la sonde 101B. À partir des profondeurs PA et PB, et connaissant la distance L séparant les sondes 101A et 101B, on peut remonter à l'angle φ d'inclinaison du vaisseau sanguin 150 par rapport à l'axe Ox, par exemple en considérant que la portion du vaisseau sanguin 150 située sous le dispositif 100 est sensiblement rectiligne. L'estimation de l'angle φ ainsi obtenue permet par exemple de compenser avantageusement l'inclinaison du vaisseau sanguin 150 lors de la mesure de la vitesse v par effet Doppler, par exemple en émettant avec la sonde centrale 101C un faisceau ultrasonore incident 201' formant un angle α' avec la normale à la surface inférieure 100I du dispositif 100. L'angle α' est par exemple ajusté de sorte que le faisceau ultrasonore 201' atteigne le vaisseau sanguin 150 sous un angle d'incidence le plus proche possible de l'angle θ (égal à l'angle θ, dans l'exemple illustré en figure 3).

L'estimation de l'angle φ, grâce aux sondes latérales 101A, 101B du dispositif 100, permet en outre de modifier une distance focale du faisceau ultrasonore incident émis par la sonde centrale 101C. Dans l'exemple représenté, le faisceau ultrasonore incident 201' est focalisé au voisinage de l'axe médian 150M du vaisseau sanguin 150, ce qui permet là encore de compenser l'inclinaison du vaisseau sanguin 150 par rapport au dispositif 100 et d'obtenir avantageusement des mesures plus précises que si la sonde 101C émettait le faisceau ultrasonore 201.

Par ailleurs, les faisceaux ultrasonores émis par les sondes latérales 101A, 101B peuvent être ajustés en fonction de la position estimée du vaisseau sanguin 150. Plus précisément, une direction d'émission et une distance focale des faisceaux ultrasonores respectivement émis par les barrettes de transducteurs ultrasonores 103A, 103B peuvent être ajustées en fonction de la position estimée du vaisseau sanguin 150. À titre de variante, une fois la position du vaisseau sanguin 150 détectée par les sondes 101A, 101B, une partie seulement des transducteurs ultrasonores 103A, 103B, par exemple le transducteur ultrasonore 103A, 103B de chaque barrette le plus proche de l'axe médian 150M, peut être utilisée pour suivre l'évolution temporelle du diamètre D.

Dans le cas où les sondes 101A, 101B mettent en œuvre le procédé de détermination de vitesse d'onde de pouls par mesure de temps de transit d'impulsion précédemment décrit en relation avec la figure 2, l'estimation de la position du vaisseau sanguin 150 par rapport au dispositif 100 peut en outre être avantageusement utilisée pour compenser une erreur de distance entre les zones de mesure du diamètre D à l'aplomb des transducteurs ultrasonores 103A, 103B. Dans l'exemple de la figure 3, ces zones sont en effet séparées par une distance L' non pas égale à la distance L entre les sondes 101A et 101B, comme dans l'exemple illustré en figure 2, mais égale à L*cos(φ).

On a illustré en figure 3 un exemple dans lequel l'axe médian 150M du vaisseau sanguin 150 est incliné d'un angle φ négatif par rapport à l'axe Ox et dans lequel le faisceau ultrasonore 201' est dirigé vers l'aval du vaisseau sanguin 150 (vers la droite, dans l'orientation de la figure 3). À titre de variante, le vaisseau sanguin 150 pourrait être incliné d'un angle φ positif par rapport à l'axe Ox, la profondeur PA étant dans ce cas supérieure à la profondeur PB. La compensation de l'inclinaison du vaisseau sanguin 150 serait alors réalisée par exemple en ajustant l'angle α' de sorte que le faisceau ultrasonore 201' atteigne le vaisseau sanguin 150 sous un angle d'incidence compris entre 120 et 150°. Dans ce cas, le faisceau ultrasonore 201' serait dirigé vers l'amont du vaisseau sanguin 150 (vers la gauche, dans l'orientation de la figure 3).

Bien que l'on ait décrit ci-dessus un exemple dans lequel le vaisseau sanguin 150 est parallèle au plan Oxz et incliné par rapport à l'angle Ox, les modes de réalisation décrits s'appliquent également à des cas dans lesquels le vaisseau sanguin 150 présente une orientation quelconque dans l'espace. À titre d'exemple, les modes de réalisation décrits sont transposables par la personne du métier à des cas dans lesquels le vaisseau sanguin 150 est parallèle au plan Oxy et incliné par rapport à l'axe Ox et à des cas dans lesquels le vaisseau sanguin 150 n'est parallèle ni au plan Oxz, ni au plan Oxy.

En outre, on a décrit ci-dessus des modes de réalisation dans lesquels la direction et/ou la distance focale des faisceaux ultrasonores émis par les barrettes de transducteurs ultrasonores 103A, 103B, 103C peuvent être ajustées en fonction de la position estimée du vaisseau sanguin 150. Toutefois, il serait également possible de tirer profit de l'estimation de la position du vaisseau sanguin 150 par rapport au dispositif 100 pour compenser des mesures effectuées par les sondes 101A, 101B, 101C sans modifier la direction et/ou la distance focale des faisceaux ultrasonores qu'elles émettent, ou en complément de telles modifications.

Par ailleurs, bien que l'on ait décrit un exemple dans lequel les deux barrettes latérales de transducteurs 103A et 103B sont utilisées pour estimer la position du vaisseau sanguin 150, on pourrait, à titre de variante, prévoir d'utiliser une seule barrette latérale de transducteurs, par exemple les transducteurs 103A de la sonde 101A ou les transducteurs 103B de la sonde 101B, pour estimer la position du vaisseau sanguin 150. Cette variante est à la portée de la personne du métier à partir des indications de la présente description.

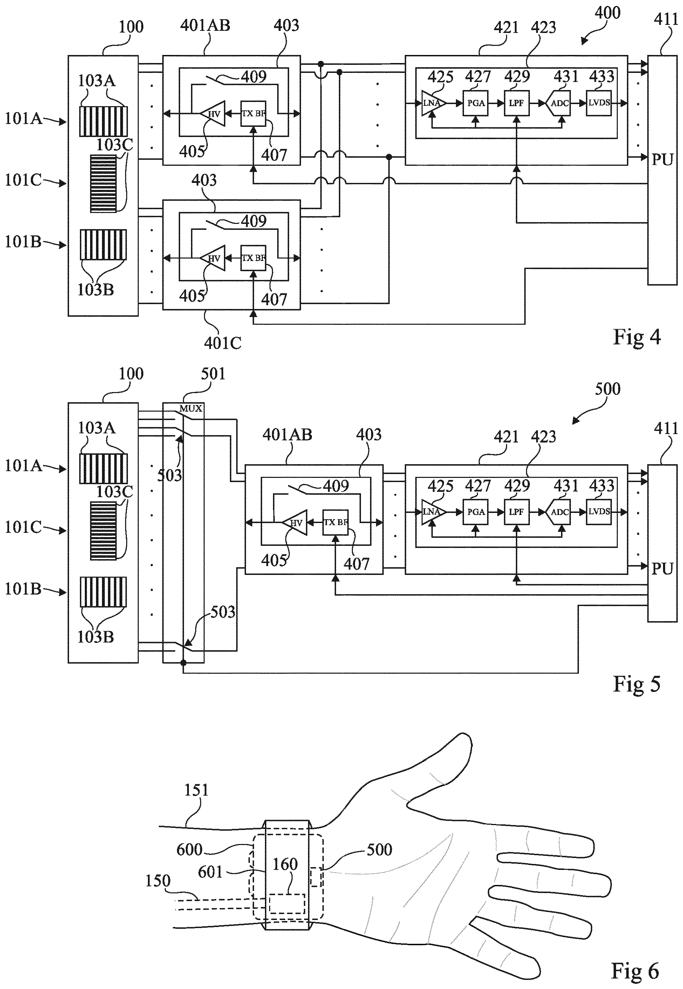

La figure 4 représente, de façon schématique et partielle, un exemple de réalisation d'un circuit de commande 400 du dispositif 100 de la figure 1.

Dans l'exemple représenté, le circuit 400 comporte deux générateurs d'impulsions 401AB, 401C ("pulser", en anglais), aussi appelés circuits d'émission. Le générateur d'impulsions 401AB est par exemple connecté aux transducteurs ultrasonores 103A, 103B des sondes latérales 101A, 101B et le générateur d'impulsions 401C est par exemple connecté aux transducteurs ultrasonores 103C de la sonde centrale 101C. Dans cet exemple, chaque générateur d'impulsions 401AB, 401C comporte, pour chaque transducteur ultrasonore 103A, 103B, 103C auquel il est connecté, une voie 403. À titre d'exemple, dans le cas où les barrettes des sondes latérales 101A, 101B comportent chacune huit transducteurs ultrasonores 103A, 103B et où la barrette de la sonde centrale 101C comporte seize transducteurs ultrasonores 103C, chaque générateur d'impulsions 401AB, 401C comporte seize voies 403 identiques, aux dispersions de fabrication près. À titre d'exemple, les générateurs d'impulsions 401AB et 401C sont identiques, aux dispersions de fabrication près.

Dans l'exemple représenté, chaque voie 403 comprend plus précisément un amplificateur 405 (HV), par exemple un amplificateur haute tension, un formeur de faisceaux d'émission 407 (TX BF) et un commutateur d'émission-réception 409. Dans l'exemple illustré en figure 4, le formeur de faisceaux d'émission 407 comporte une entrée connectée à une sortie d'une unité de traitement 411 (PU), par exemple une matrice prédiffusée programmable ("Field-Programmable Gate Array" - FPGA, en anglais), et une sortie connectée à une entrée de l'amplificateur haute-tension 405. L'amplificateur 405 comporte une sortie connectée à l'une des bornes du commutateur 409 et à l'un des transducteurs ultrasonores 103A, 103B, 103C. Dans cet exemple, l'autre borne de chaque commutateur 409 est connectée à un circuit frontal analogique 421 ("analog front end", en anglais), aussi appelé circuit de réception.

Le circuit de réception 421 comprend une pluralité de voies de réception 423. Dans cet exemple, le circuit 421 comporte un nombre de voies de réception 423 égal à la moitié du nombre de transducteurs ultrasonores 103A, 103B, 103C du dispositif 100. À titre d'exemple, dans le cas où les barrettes des sondes latérales 101A, 101B comportent chacune huit transducteurs ultrasonores 103A, 103B et où la barrette de la sonde centrale 101C comporte seize transducteurs ultrasonores, le circuit 421 comporte seize voies 423 identiques, aux dispersions de fabrication près.

Dans l'exemple représenté, chaque voie 423 comprend plus précisément un amplificateur 425 (LNA), par exemple un amplificateur à faible bruit, un amplificateur 427 (PGA), par exemple un amplificateur à gain programmable, un filtre passe-bas 429 (LPF), un convertisseur analogique-numérique 431 (ADC) et un convertisseur série 433 (LVDS), par exemple un convertisseur capable de fonctionner en signalisation différentielle basse tension. Dans cet exemple, l'amplificateur 425 de chaque voie 423 comporte une entrée connectée à l'une des voies 403 du générateur d'impulsions 401AB et à l'une des voies 403 du générateur d'impulsions 401C, et une sortie connectée à une entrée de l'amplificateur 427. L'amplificateur 427 comporte une sortie connectée à une entrée du filtre 429. Le convertisseur 431 comprend une entrée connectée au filtre 429 et une sortie connectée à une entrée du convertisseur série 433. Dans l'exemple représenté, les amplificateurs 425 et 427, le filtre 429 et le convertisseur 431 comportent chacun une entrée de commande connectée à une sortie de l'unité de traitement 411. En outre, chaque voie 423 du circuit 421 comporte une sortie, correspondant à une sortie du convertisseur série 433, connectée à l'unité de traitement 411.

Un exemple de fonctionnement du dispositif 100 associé au circuit 400 va maintenant être décrit en relation avec la figure 4.

Au cours d'une première étape d'émission, les commutateurs 409 du générateur d'impulsions 401AB sont par exemple tous à l'état ouvert et des faisceaux ultrasonores sont émis par les sondes latérales 101A et 101B. Pendant la première étape d'émission, l'unité de traitement 411 est par exemple configurée pour ne transmettre aucun signal aux formeurs de faisceaux d'émission 407 du générateur d'impulsions 401C, la sonde 101C n'émettant alors par exemple aucun faisceau ultrasonore. Puis, lors d'une première étape de réception postérieure à la première étape d'émission, on ferme par exemple tous les commutateurs 409 du générateur d'impulsions 401AB afin de permettre aux sondes latérales 101A, 101B de capter des échos ultrasonores. Dans cet exemple, la position du vaisseau sanguin 150 par rapport au dispositif 100 est par exemple estimée à l'issue de la première étape de réception.

Ensuite, lors d'une deuxième étape d'émission postérieure à la première étape de réception, tous les commutateurs 409 du générateur d'impulsions 401C sont à l'état ouvert et des faisceaux ultrasonores sont émis par la sonde centrale 101C. Pendant la deuxième étape d'émission, l'unité de traitement 411 est par exemple configurée pour ne transmettre aucun signal aux formeurs de faisceaux d'émission 407 du générateur d'impulsions 401AB, les sondes 101A et 101B n'émettant alors par exemple aucun faisceau ultrasonore. Enfin, lors d'une deuxième étape de réception postérieure à la deuxième étape d'émission, on ferme par exemple tous les commutateurs 409 du générateur d'impulsions 401C afin de permettre à la sonde centrale 101C de capter des échos ultrasonores. La mesure du débit Dᵥ est par exemple réalisée à l'issue de la deuxième étape de réception.

Une fois la deuxième étape de réception achevée, on procède par exemple à l'ajustement de paramètres d'émission du faisceau émis par la sonde centrale 101C et/ou des faisceaux émis par les sondes 101A, 101B en fonction de la position du vaisseau sanguin 150 par rapport au dispositif 100 telle qu'estimée lors de la première étape de réception. On peut ensuite par exemple répéter les étapes d'émission et de réception précédemment décrites en utilisant les paramètres d'émission modifiés.

À titre d'exemple, la position du vaisseau sanguin 150 est estimée et les paramètres d'émission de la sonde 101C et/ou des sondes 101A, 101B sont modifiés au début d'une phase d'évaluation de l'activité cardiovasculaire d'un patient, les paramètres d'émission de la sonde 101C et/ou des sondes 101A, 101B demeurant par la suite inchangés jusqu'à la prochaine phase d'évaluation, par exemple réalisée sur un autre patient. À titre de variante, on peut prévoir que les opérations d'estimation de la position du vaisseau sanguin 150 et d'ajustement des paramètres d'émission de la sonde 101C et/ou des sondes 101A, 101B soient répétées au cours d'une même phase d'évaluation de l'activité cardiovasculaire du patient.

La figure 5 représente, de façon schématique et partielle, un exemple de réalisation d'un circuit de commande 500 du dispositif 100 de la figure 1. Le circuit 500 de la figure 5 comporte des éléments communs avec le circuit 400 de la figure 4.

Le circuit 500 de la figure 5 diffère du circuit 400 de la figure 4 principalement en ce que, dans l'exemple représenté, le circuit 500 comporte un seul générateur d'impulsions, par exemple le générateur d'impulsions 401AB précédemment décrit en relation avec la figure 4. En outre, le circuit 500 diffère du circuit 400 en ce qu'il comporte un multiplexeur 501 (MUX) reliant les barrettes de transducteurs ultrasonores 103A, 103B, 103C des sondes 101A, 101B, 101C du dispositif 100 au générateur d'impulsions 401AB. Dans l'exemple représenté où le dispositif comprend trente-deux transducteurs ultrasonores 103A, 103B, 103C, le multiplexeur 501 comporte plus précisément seize voies et est de type 1 vers 2.

À titre d'exemple, chaque voie du multiplexeur 501 comprend, comme dans l'exemple illustré en figure 5, un commutateur 503 de type unipolaire bidirectionnel ("Single Pole Double Throw" - SPDT, en anglais) dont une borne d'entrée est connectée à l'une des voies 403 du générateur d'impulsions 401AB, dont une première borne de sortie est par exemple connectée à l'un des transducteurs ultrasonores 103A, 103B des sondes latérales 101A, 101B et dont une deuxième borne de sortie est par exemple connectée à l'un des transducteurs ultrasonores 103C de la sonde centrale 101C. Le multiplexeur 501 comporte en outre une entrée de commande connectée à l'unité de traitement 411 et permettant par exemple de faire commuter tous les commutateurs 503 entre leurs première et deuxième bornes de sortie, par exemple de façon sensiblement simultanée. À titre d'exemple, l'entrée de commande reçoit de l'unité de traitement 411 un signal de commande dont un premier niveau place tous les commutateurs 503 dans un état où leur borne d'entrée est connectée à leur première borne de sortie et dont un deuxième niveau place tous les commutateurs 503 dans un autre état où leur borne d'entrée est connectée à leur deuxième borne de sortie.

Un exemple de fonctionnement du dispositif 100 associé au circuit 500 va maintenant être décrit en relation avec la figure 5.

Au cours d'une première étape d'émission, les commutateurs 409 du générateur d'impulsions 401AB sont par exemple tous à l'état ouvert, les interrupteurs 503 du multiplexeur 501 sont par exemple placés dans l'état dans lequel leur borne d'entrée est connectée à leur première borne de sortie, connectée aux transducteurs ultrasonores 103A, 103B dans cet exemple, et des faisceaux ultrasonores sont émis par les sondes latérales 101A et 101B. Puis, lors d'une première étape de réception postérieure à la première étape d'émission, on ferme par exemple tous les commutateurs 409 du générateur d'impulsions 401AB afin de permettre aux sondes latérales 101A, 101B de capter des échos ultrasonores, les interrupteurs 503 du multiplexeur 501 demeurant dans l'état où leur borne d'entrée est connectée à leur première borne de sortie. Dans cet exemple, la position du vaisseau sanguin 150 par rapport au dispositif 100 est par exemple estimée à l'issue de la première étape de réception.

Ensuite, lors d'une deuxième étape d'émission postérieure à la première étape de réception, les commutateurs 409 du générateur d'impulsions 401AB sont par exemple ouverts, les interrupteurs 503 du multiplexeur 501 sont par exemple commutés vers l'état dans lequel leur borne d'entrée est connectée à leur deuxième borne de sortie, connectée aux transducteurs ultrasonores 103C dans cet exemple, et des faisceaux ultrasonores sont émis par la sonde centrale 101C. Enfin, lors d'une deuxième étape de réception postérieure à la deuxième étape d'émission, on ferme par exemple tous les commutateurs 409 du générateur d'impulsions 401AB afin de permettre à la sonde centrale 101C de capter des échos ultrasonores, les interrupteurs 503 du multiplexeur 501 demeurant dans l'état où leur borne d'entrée est connectée à leur deuxième borne de sortie, afin de permettre à la sonde centrale 101C de capter des échos ultrasonores. La mesure du débit Dᵥ est par exemple réalisée à l'issue de la deuxième étape de réception.

Une fois la deuxième étape de réception achevée, on procède par exemple à l'ajustement de paramètres d'émission du faisceau émis par la sonde centrale 101C et/ou des faisceaux émis par les sondes 101A, 101B en fonction de la position estimée du vaisseau sanguin 150 par rapport au dispositif 100. On peut ensuite par exemple répéter les étapes d'émission et de réception précédemment décrites en utilisant les paramètres d'émission modifiés.

Un avantage des circuits 400, 500 précédemment décrits tient au fait qu'ils présentent un faible nombre de composants électroniques et de voies, ce qui rend les circuits 400, 500 et le dispositif 100 compatibles avec une intégration dans un dispositif portable et présentant un coût d'acquisition peu élevé.

La figure 6 illustre un exemple d'intégration du dispositif 100 de la figure 1 dans un dispositif 600 de type bracelet porté au bras 151 d'un utilisateur, par exemple une montre intelligente. Dans l'exemple représenté, le dispositif 600 intègre en outre le circuit de commande 500 du dispositif 100. Le dispositif 100 est par exemple situé dans une zone du dispositif 600 destinée à être placée sensiblement en regard de l'artère ulnaire 150 du bras 151 de l'utilisateur. À titre d'exemple, le dispositif 100 est intégré dans un bracelet 601 du dispositif 600.

Divers modes de réalisation et variantes ont été décrits. La personne du métier comprendra que certaines caractéristiques de ces divers modes de réalisation et variantes pourraient être combinées, et d'autres variantes apparaîtront à la personne du métier. En particulier, la personne du métier est capable de substituer le circuit de commande 500 du dispositif 600 de la figure 6 par le circuit de commande 400 précédemment décrit en relation avec la figure 4.

Enfin, la mise en œuvre pratique des modes de réalisation et variantes décrits est à la portée de la personne du métier à partir des indications fonctionnelles données ci-dessus. En particulier, la réalisation pratique du dispositif 100, notamment la géométrie, le positionnement et la commande des transducteurs ultrasonores 103A, 103B, 103C des sondes 101A, 101B, 101C du dispositif 100, et le pilotage des éléments des circuits 400, 500 par l'unité de traitement 411 sont à la portée de la personne du métier à partir des indications de la présente description.

## Revendications

1. Dispositif électronique d'évaluation d'activité cardiovasculaire humaine, le dispositif étant portable et présentant des dimensions extérieures compatibles avec un port à un poignet d'un utilisateur, le dispositif comprenant des première et deuxième sondes (101A, 101B) sensiblement parallèles entre elles et situées de part et d'autre d'une troisième sonde (101C), sensiblement perpendiculaire aux première et deuxième sondes, chaque sonde (101A, 101B, 101C) comportant une barrette de transducteurs ultrasonores (103A, 103B, 103C), et un circuit de commande (400 ; 500) comprenant une unité de traitement (411), le circuit de commande étant configuré pour, au moyen de l'unité de traitement (411) :
a) estimer, par un premier faisceau ultrasonore émis par la première sonde (101A), une position d'un vaisseau sanguin (150) par rapport au dispositif ; et
b) ajuster un deuxième faisceau ultrasonore (201 ; 201'), émis par la troisième sonde (101C), en fonction de la position estimée du vaisseau sanguin (150).

2. Dispositif selon la revendication 1, dans lequel le circuit de commande est configuré pour, à l'étape b), ajuster une direction d'émission du deuxième faisceau ultrasonore (201 ; 201') en fonction de la position estimée du vaisseau sanguin (150).

3. Dispositif selon la revendication 1 ou 2, dans lequel le circuit de commande est en outre configuré pour, à l'étape b), ajuster une distance focale du deuxième faisceau ultrasonore (201 ; 201') en fonction de la position estimée du vaisseau sanguin (150).

4. Dispositif selon l'une quelconque des revendications 1 à 3, dans lequel le circuit de commande est configuré pour ajuster le premier faisceau ultrasonore émis par la première sonde (101A) en fonction de la position estimée du vaisseau sanguin (150).

5. Dispositif selon l'une quelconque des revendications 1 à 4, dans lequel la deuxième sonde (101B) est configurée pour émettre un troisième faisceau ultrasonore.

6. Dispositif selon la revendication 5, dans lequel le circuit de commande est en outre configuré pour, au cours de l'étape a), estimer la position du vaisseau sanguin (150) par rapport au dispositif au moyen du troisième faisceau ultrasonore émis par la deuxième sonde (101B).

7. Dispositif selon la revendication 5 ou 6, dans lequel le circuit de commande est configuré pour estimer une vitesse d'onde de pouls à l'intérieur du vaisseau sanguin (150) à partir des premier et troisième faisceaux ultrasonores émis respectivement par les première et deuxième sondes (101A, 101B).

8. Dispositif selon l'une quelconque des revendications 1 à 7, dans lequel le circuit de commande est en outre configuré pour estimer le débit sanguin à l'intérieur du vaisseau sanguin (150), à partir du deuxième faisceau ultrasonore (201 ; 201'), par effet Doppler.

9. Dispositif selon l'une quelconque des revendications 1 à 8, dans lequel le vaisseau sanguin (150) est l'artère ulnaire.

10. Dispositif selon l'une quelconque des revendications 1 à 9, dans lequel le circuit de commande (400) comprend un premier générateur d'impulsions (401AB) connecté aux transducteurs ultrasonores (103A, 103B) des barrettes des première et deuxième sondes (101A, 101B) et un deuxième générateur d'impulsions (401C) connecté aux transducteurs ultrasonores (103C) de la barrette de la troisième sonde (101C).

11. Dispositif selon l'une quelconque des revendications 1 à 9, dans lequel le circuit de commande (500) comprend un seul générateur d'impulsions (401AB) relié, par l'intermédiaire d'un multiplexeur (501), aux transducteurs ultrasonores (103A, 103B, 103C) des barrettes des première, deuxième et troisième sondes (101A, 101B, 101C).

12. Dispositif selon la revendication 11, dans lequel la barrette de transducteurs ultrasonores (103C) de la troisième sonde (101C) comporte deux fois plus de transducteurs ultrasonores (103C) que chacune des barrettes de transducteurs ultrasonores (103A, 103B) des première et deuxième sondes (101A, 101B), le multiplexeur (501) étant un multiplexeur 1 vers 2.

13. Montre (600) ou bracelet connecté comportant un dispositif selon l'une quelconque des revendications 1 à 12.

14. Procédé d'évaluation d'activité cardiovasculaire humaine, par un dispositif électronique portable et présentant des dimensions extérieures compatibles avec un port à un poignet d'un utilisateur et comprenant des première et deuxième sondes (101A, 101B) sensiblement parallèles entre elles et situées de part et d'autre d'une troisième sonde (101C), sensiblement perpendiculaire aux première et deuxième sondes, chaque sonde (101A, 101B, 101C) comportant une barrette de transducteurs ultrasonores (103A, 103B, 103C), et un circuit de commande (400 ; 500) comprenant une unité de traitement (411), le procédé comprenant les étapes suivantes, mises en œuvre par le circuit de commande au moyen de l'unité de traitement (411) :
a) estimer, par un premier faisceau ultrasonore émis par la première sonde (101A), une position d'un vaisseau sanguin (150) par rapport au dispositif ; et
b) ajuster un deuxième faisceau ultrasonore (201 ; 201'), émis par la troisième sonde (101C), en fonction de la position estimée du vaisseau sanguin (150).

15. Procédé selon la revendication 14, dans lequel, à l'étape a), la position du vaisseau sanguin (150) par rapport au dispositif est estimée en outre par un troisième faisceau ultrasonore émis par la deuxième sonde (101B).

## Patentansprüche

1. Elektronische Vorrichtung zur Beurteilung einer kardiovaskulären Aktivität eines Menschen, wobei die Vorrichtung tragbar ist und Außenabmessungen aufweist, die mit dem Tragen am Handgelenk eines Benutzers kompatibel sind, wobei die Vorrichtung eine erste und eine zweite Sonde (101A, 101B) aufweist, die im Wesentlichen parallel zueinander sind und die sich auf beiden Seiten einer dritten Sonde (101C) befinden, die im Wesentlichen senkrecht zur ersten und zweiten Sonde ist, wobei jede Sonde (101A, 101B, 101C) eine Anordnung von Ultraschallwandlern (103A, 103B, 103C) und eine Steuerschaltung (400; 500) mit einer Verarbeitungseinheit (411) aufweist, wobei die Steuerschaltung unter Verwendung der Verarbeitungseinheit (411) konfiguriert ist zum:
a) Schätzen, anhand eines von der ersten Sonde (101A) emittierten ersten Ultraschallstrahls, einer Position eines Blutgefäßes (150) in Bezug auf die Vorrichtung; und
b) Anpassen eines von der dritten Sonde (101C) emittierten zweiten Ultraschallstrahls (201; 201') entsprechend der geschätzten Position des Blutgefäßes (150).

2. Vorrichtung nach Anspruch 1, wobei die Steuerschaltung so konfiguriert ist, dass sie in Schritt b) eine Emissionsrichtung des zweiten Ultraschallstrahls (201; 201') entsprechend der geschätzten Position des Blutgefäßes (150) anpasst.

3. Vorrichtung nach Anspruch 1 oder 2, wobei die Steuerschaltung ferner so konfiguriert ist, dass sie in Schritt b) eine Brennweite des zweiten Ultraschallstrahls (201; 201') entsprechend der geschätzten Position des Blutgefäßes (150) anpasst.

4. Vorrichtung nach einem der Ansprüche 1 bis 3, wobei die Steuerschaltung so konfiguriert ist, dass sie den von der ersten Sonde (101A) emittierten ersten Ultraschallstrahl entsprechend der geschätzten Position des Blutgefäßes (150) anpasst.

5. Vorrichtung nach einem der Ansprüche 1 bis 4, wobei die zweite Sonde (101B) so konfiguriert ist, dass sie einen dritten Ultraschallstrahl emittiert.

6. Vorrichtung nach Anspruch 5, wobei die Steuerschaltung ferner so konfiguriert ist, dass sie, während Schritt a), die Position des Blutgefäßes (150) in Bezug auf die Vorrichtung mittels des von der zweiten Sonde (101B) emittierten dritten Ultraschallstrahls schätzt.

7. Vorrichtung nach Anspruch 5 oder 6, wobei die Steuerschaltung so konfiguriert ist, dass sie eine Pulswellengeschwindigkeit innerhalb des Blutgefäßes (150) auf Grundlage des ersten und des dritten Ultraschallstrahls schätzt, die jeweils von der ersten und zweiten Sonde (101A, 101B) emittiert werden.

8. Vorrichtung nach einem der Ansprüche 1 bis 7, wobei die Steuerschaltung ferner so konfiguriert ist, dass sie die Blutflussrate innerhalb des Blutgefäßes (150) auf Grundlage des zweiten Ultraschallstrahls (201; 201') durch den Doppler-Effekt schätzt.

9. Vorrichtung nach einem der Ansprüche 1 bis 8, wobei das Blutgefäß (150) die Arteria ulnaris ist.

10. Vorrichtung nach einem der Ansprüche 1 bis 9, wobei die Steuerschaltung (400) einen ersten Impulsgeber (401AB) aufweist, der mit den Ultraschallwandlern (103A, 103B) der Anordnungen der ersten und zweiten Sonde (101A, 101B) verbunden ist, und einen zweiten Impulsgeber (401C), der mit den Ultraschallwandlern (103C) der Anordnung der dritten Sonde (101C) verbunden ist.

11. Vorrichtung nach einem der Ansprüche 1 bis 9, wobei die Steuerschaltung (500) einen einzigen Impulsgeber (401AB) aufweist, der über einen Multiplexer (501) mit den Ultraschallwandlern (103A, 103B, 103C) der Anordnungen der ersten, zweiten und dritten Sonde (101A, 101B, 101C) gekoppelt ist.

12. Vorrichtung nach Anspruch 11, wobei die Anordnung von Ultraschallwandlern (103C) der dritten Sonde (101C) doppelt so viele Ultraschallwandler (103C) aufweist wie jede der Anordnungen von Ultraschallwandlern (103A, 103B) der ersten und zweiten Sonde (101A, 101B), wobei der Multiplexer (501) ein 1-zu-2-Multiplexer ist.

13. Vernetzte Uhr (600) oder Armband, das eine Vorrichtung nach einem der Ansprüche 1 bis 12 aufweist.

14. Verfahren zur Beurteilung einer kardiovaskulären Aktivität eines Menschen durch eine elektronische die Vorrichtung, die tragbar ist und die Außenabmessungen aufweist, die mit dem Tragen am Handgelenk eines Benutzers kompatibel sind, und das eine erste und eine zweite Sonde (101A, 101B) aufweist, die im Wesentlichen parallel zueinander und auf beiden Seiten einer dritten Sonde (101C) angeordnet sind, die im Wesentlichen senkrecht zur ersten und zweiten Sonde ist, wobei jede Sonde (101A, 101B, 101C) eine Anordnung von Ultraschallwandlern (103A, 103B, 103C) und eine Steuerschaltung (400; 500) mit einer Verarbeitungseinheit (411) aufweist, wobei das Verfahren die folgenden Schritte aufweist, die von der Steuerschaltung unter Verwendung der Verarbeitungseinheit (411) ausgeführt werden:
a) Schätzen, anhand eines von der ersten Sonde (101A) emittierten ersten Ultraschallstrahls, einer Position eines Blutgefäßes (150) in Bezug auf die Vorrichtung; und
b) Anpassen eines von der dritten Sonde (101C) emittierten zweiten Ultraschallstrahls (201; 201') entsprechend der geschätzten Position des Blutgefäßes (150).

15. Verfahren nach Anspruch 14, wobei, in Schritt a), die Position des Blutgefäßes (150) in Bezug auf die Vorrichtung zusätzlich durch einen dritten Ultraschallstrahl geschätzt wird, der von der zweiten Sonde (101B) emittiert wird.

## Claims

1. Electronic device for assessing a human cardiovascular activity, the device being portable and having outer dimensions compatible with its being worn on a user's wrist, the device comprising first and second probes (101A, 101B) substantially parallel to each other and located on either side of a third probe (101C), substantially perpendicular to the first and second probes, each probe (101A, 101B, 101C) comprising an array of ultrasound transducers (103A, 103B, 103C), and a control circuit (400; 500) comprising a processing unit (411), the control circuit being configured to, using the processing unit (411):
a) estimate, by means of a first ultrasound beam emitted by the first probe (101A), a position of a blood vessel (150) with respect to the device; and
b) adjust a second ultrasound beam (201; 201'), emitted by the third probe (101C), according to the estimated position of the blood vessel (150).

2. Device according to claim 1, wherein the control circuit is configured to, at step b), adjust a direction of emission of the second ultrasound beam (201; 201') according to the estimated position of the blood vessel (150).

3. Device according to claim 1 or 2, wherein the control circuit is further configured to, at step b), adjust focal distance of the second ultrasound beam (201; 201') according to the estimated position of the blood vessel (150).

4. Device according to any of claims 1 to 3, wherein the control circuit is configured to adjust the first ultrasound beam emitted by the first probe (101A) according to the estimated position of the blood vessel (150).

5. Device according to any of claims 1 to 4, wherein the second probe (101B) is configured to emit a third ultrasound beam.

6. Device according to claim 5, wherein the control circuit is further configured to, during step a), estimate the position of the blood vessel (150) with respect to the device by means of the third ultrasound beam emitted by the second probe (101B).

7. Device according to claim 5 or 6, wherein the control circuit is configured to estimate a pulse wave velocity inside of the blood vessel (150) based on the first and third ultrasound beams respectively emitted by the first and second probes (101A, 101B).

8. Device according to any of claims 1 to 7, wherein the control circuit is further configured to estimate the blood flow rate inside of the blood vessel (150), based on the second ultrasound beam (201; 201'), by Doppler effect.

9. Device according to any of claims 1 to 8, wherein the blood vessel (150) is the ulnar artery.

10. Device according to any of claims 1 to 9, wherein the control circuit (400) comprises a first pulser (401AB) connected to the ultrasound transducers (103A, 103B) of the arrays of the first and second probes (101A, 101B) and a second pulser (401C) connected to the ultrasound transducers (103C) of the array of the third probe (101C).

11. Device according to any of claims 1 to 9, wherein the control circuit (500) comprises a single pulser (401AB) coupled, via a multiplexer (501), to the ultrasound transducers (103A, 103B, 103C) of the arrays of the first, second, and third probes (101A, 101B, 101C).

12. Device according to claim 11, wherein the array of ultrasound transducers (103C) of the third probe (101C) comprises twice more ultrasound transducers (103C) than each of the arrays of ultrasound transducers (103A, 103B) of the first and second probes (101A, 101B), the multiplexer (501) being a 1-to-2 multiplexer.

13. Connected watch (600) or bracelet comprising a device according to any of claims 1 to 12.

14. Method of assessment of a human cardiovascular activity, by an electronic device portable and having outer dimensions compatible with its being worn on a user's wrist and comprising first and second probes (101A, 101B) substantially parallel to each other and located on either side of a third probe (101C), substantially perpendicular to the first and second probes, each probe (101A, 101B, 101C) comprising an array of ultrasound transducers (103A, 103B, 103C), and a control circuit (400; 500) comprising a processing unit (411), the method comprising the following steps, implemented by the control circuit using the processing unit (411):
a) estimate, by means of a first ultrasound beam emitted by the first probe (101A), a position of a blood vessel (150) with respect to the device; and
b) adjust a second ultrasound beam (201; 201'), emitted by the third probe (101C), according to the estimated position of the blood vessel (150).

15. Method according to claim 14, wherein, at step a), the position of the blood vessel (150) with respect to the device is further estimated by a third ultrasound beam emitted by the second probe (101B).
